(19) Europäisches Patentamt
European Patent Office
Office européen des brevets

(11) **EP 3 659 154 B1**

(12) **EUROPEAN PATENT SPECIFICATION**

(45) Date of publication and mention
of the grant of the patent:
**07.09.2022 Bulletin 2022/36**

(21) Application number: **18743026.9**

(22) Date of filing: **25.07.2018**

(51) International Patent Classification (IPC):
***G16H 50/30*** (2018.01)

(52) Cooperative Patent Classification (CPC):
**G16H 50/30**

(86) International application number:
**PCT/EP2018/070188**

(87) International publication number:
**WO 2019/020697 (31.01.2019 Gazette 2019/05)**

(54) **A SYSTEM AND METHOD FOR DETERMINING A RISK LEVEL OF A POLLEN-INDUCED ALLERGY OF A USER**

SYSTEM UND VERFAHREN ZUR BESTIMMUNG EINES RISIKONIVEAUS EINER POLLENINDUZIERTEN ALLERGIE EINES BENUTZERS

SYSTÈME ET PROCÉDÉ DE DÉTERMINATION D'UN NIVEAU DE RISQUE D'UNE ALLERGIE D'UN UTILISATEUR INDUITE PAR UN POLLEN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.07.2017 PCT/CN2017/094206
19.10.2017 EP 17197197**

(43) Date of publication of application:
**03.06.2020 Bulletin 2020/23**

(73) Proprietor: **Koninklijke Philips N.V.
5656 AG Eindhoven (NL)**

(72) Inventors:
• **KONG, Tao
5656 AE Eindhoven (NL)**
• **CHEN, Shuang
5656 AE Eindhoven (NL)**

(74) Representative: **de Vries, Janna et al
Philips Domestic Appliances Nederland B.V.
High Tech Campus 42
5656 AE Eindhoven (NL)**

(56) References cited:
**WO-A1-2017/148876      US-A1- 2015 242 586**

• **EDMUND Y W SETO ET AL: "A wireless body sensor network for the prevention and management of asthma", INDUSTRIAL EMBEDDED SYSTEMS, 2009. SIES '09. IEEE INTERNATIONAL SYMPOSIUM ON, IEEE, PISCATAWAY, NJ, USA, 8 July 2009 (2009-07-08), pages 120-123, XP031504609, ISBN: 978-1-4244-4109-9**

**Description**

FIELD OF THE INVENTION

This invention relates to a system for communicating to a user a risk level of a pollen-induced allergy (i.e. allergic reaction) in a location, thus allowing preventative measures to be taken. In particular, the invention relates to a system having inputs for receiving information such as that relating to the pollen level in the location. This information is used in order to determine the risk level.

BACKGROUND OF THE INVENTION

Pollen is produced by trees, flowers, grasses, and weeds in order to fertilize other plants of the same species. It is one of the most common triggers of seasonal allergies for people. Many people have the potential to suffer an adverse immune response when they breathe in pollen, resulting in pollen allergy. In people with pollen allergies, the immune system mistakenly identifies the harmless pollen as a dangerous intruder and begins to produce chemicals to fight against the pollen. More specifically, pollen carries allergens, and when the pollen is inhaled into the nose, it can release the allergen which is responsible for inducing the allergic symptoms.

The most common symptoms caused by pollen include nasal congestion, runny nose, itchy, watery eyes, scratchy throat, cough, swollen, and bluish-colored skin beneath the eyes. Pollen can also trigger allergic rhinitis (also known as hay fever). Moderate or severe allergic rhinitis can lead to more frequent sinus infections and therefore causes considerable impairment in quality of life. Furthermore, pollen can even cause asthma, which is a long-term inflammatory disease of the airways of the lungs and could possibly induce death if no timely treatment is provided.

These symptoms arise when a pollen threshold value for symptom development of an individual is surpassed. Aside from the pollen level itself, a number of other factors can influence the pollen threshold value for symptom development. These include environmental factors such as the season and weather. The characteristics of the individual also influence this threshold value.

It is therefore desirable that people liable to pollen-induced allergies are provided with information on the risk-level of such allergies in a location. If this risk level is deemed too high, preventative measures can be taken, such as avoiding that location or carrying suitable medication.

Systems for communicating a risk level of a pollen-induced allergy of a user are known. For example, pollen concentration mapping and forecasts are available. An individual can consult these data and take appropriate measures. However, the information provided in such maps and forecasts is quite broad. It is also not tailored to the specific characteristics of an individual.

US 2001/029535 discloses a system for providing environmental information to users relating to the pollen level. The environmental information is measured automatically by a plurality of sensors arranged at a plurality of regions. This environmental information is processed at a base device, taking into account of user information of an individual. This processed environmental information is provided from the base device to the individual user through a network connecting the users and the base device.

US 2015/242586 discloses a system for aggregating user data and environmental data to generate a risk score of experiencing symptoms of a medical condition such as allergy and asthma symptoms.

Although this system takes into account information which is specific to the individual, the environmental information which is processed is restricted to the pollen level and therefore fairly basic.

SUMMARY OF THE INVENTION

The invention is defined by the claims.

In this system, information relating to a particulate matter level in a location is received and used in determining the risk level, along with information relating to a pollen level in a location and information relating to a sensitivity of a user to pollen allergen. Particulate matter, such as diesel exhaust particles (DEP), are found in increased levels in polluted locations. Particulate matter can promote the release of allergens from pollen. Further, the free allergen can attach onto the surface of the particulate matter. Carried by the particulate matter in this way, the pollen allergen can be breathed in, thus increasing the risk of inducing allergic symptoms.

By taking into account this information as well as information relating to a pollen level and a sensitivity of a user to pollen allergen, the risk level which is communicated to the user is therefore more accurate.

The system may comprise a pollen sensor for generating the information relating to the pollen level in the location. The use of a pollen sensor allows for the real-time measurement of the information relating to the pollen level in the location. This increases the accuracy of the risk level which is communicated to the user.

The system may comprise a particle sensor for generating the information relating to a particulate matter level in the location. Again, using a sensor allows for the real-time measurement of the information and increases the accuracy of the risk level which is communicated to the user.

The system may comprise a breathing sensor for determining a breathing rate of the user, and the processor may be further adapted to take account of the breathing rate in determining the risk level.

The risk level of a pollen-induced allergy is influenced by the breathing rate of the user, because a higher breathing rate will mean that more pollen and/or allergen is inhaled. Measuring the breathing rate and taking account of this information therefore increases the accuracy of the risk level which is communicated to the user.

The system may comprise a further input for receiving information relating to the probability of allergen release from the pollen, and the processor may be further adapted to take account of the probability of allergen release in determining the risk level.

Pollen carries allergens, and when inhaled, it can release the allergen to induce the allergic symptoms. Furthermore, when travelling in the air, pollen may also release allergen into the surrounding air, which in turn can be carried by particulate matter. The probability of allergen release from the pollen therefore influences the risk level of a pollen-induced allergy. Receiving and taking account of this information therefore increases accuracy when determining the risk.

The system comprises a further input for receiving information relating to the probability of pollen allergen binding to the particulate matter, and the processor is further adapted to take account of the probability of pollen allergen binding in determining the risk level.

Pollen allergen which has been released from pollen can be subsequently carried by particulate matter, which can in turn be breathed in by a user. The pollen allergen binding to the particulate matter therefore influences the risk level of a pollen-induced allergy. In this way, receiving and taking account of this information increases accuracy when determining the risk.

The processor may be adapted to determine the risk level based on a pollen concentration, breathing characteristics of the user, a probability of allergen release from the pollen, a probability of free allergen release, a sensitivity of the user to pollen allergen, a particulate matter concentration, and a probability that pollen allergens bind to the particulate matter.

Each of these parameters influences the risk level of a pollen-induced allergy of the user. By taking each parameter into account when determining the risk, accuracy is therefore increased.

The processor may be adapted to determine the risk level as the sum of two components $R_d$ and $R_{id}$;

wherein $R_d$ is the direct risk calculated according to the following formula:

$$R_d = \int_0^t (1 + \eta_a \cdot \eta_{fa}) \cdot C_p(t) \cdot V_c(t) \cdot \varphi \cdot dt \ .$$

wherein $R_{id}$ is the indirect risk calculated according to the following formula:

$$R_{id} = \int_0^t \sum [C_{PMi}(t) \cdot \eta_{bi}] \cdot V_c(t) \cdot \varphi \cdot dt \ ;$$

wherein

$C_p(t)$ is the pollen concentration over time,
$V_c(t)$ is the breath volume of the user over time,
$\eta_a$ is the probability of allergen release from the pollen,
$\eta_{fa}$ is the probability of free allergen release,
$\phi$ is the sensitivity of the user to pollen allergen,
$t$ is the exposure time,
$C_{PMi}(t)$ is the concentration of particles with diameter below i over time, and
$\eta_{bi}$ is the probability that pollen allergens bind to these particles.

The direct risk is therefore mediated largely by the pollen concentration, and the associated probability of allergen release from the pollen and probability of free allergen release. The indirect risk is mediated largely by the particulate matter level, and the associated probability that pollen allergens bind to these particles. Both the direct and indirect risk are mediated by the breathing rate and sensitivity of the user to pollen allergen. By taking into account both the direct and indirect risk, the overall risk level is calculated to a high degree of accuracy.

Examples in accordance with another aspect of the invention provide a method for determining a risk level of a pollen-induced allergy of a user, as defined by claim 8.

The method may comprise determining a breathing rate of the user, and taking account of the breathing rate in determining the risk level.

The method may comprise receiving information relating to the probability of allergen release from the pollen, and taking account of the probability in determining the risk level.

The method comprises receiving information relating to the probability of pollen allergen binding to the particulate matter, and taking account of the probability in determining the risk level.

The method may comprise determining the risk level based on a pollen concentration, breathing characteristics of the user, a probability of allergen release from the pollen, a probability of free allergen release, a sensitivity of the user to pollen allergen, a particulate matter concentration, and a probability that pollen allergens bind to the particulate matter.

The method may comprise determining the risk level as sum of two components $R_d$ and $R_{id}$;

wherein $R_d$ is the direct risk calculated according to the following formula:

$$R_d = \int_0^t (1 + \eta_a \cdot \eta_{fa}) \cdot C_p(t) \cdot V_c(t) \cdot \varphi \cdot dt$$

;

wherein $R_{id}$ is the indirect risk calculated according to the following formula:

$$R_{id} = \int_0^t \sum [C_{PMi}(t) \cdot \eta_{bi}] \cdot V_c(t) \cdot \varphi \cdot dt$$

;

wherein the parameters are explained above.

The processing of data may be carried out by a computer program.

BRIEF DESCRIPTION OF THE DRAWINGS

Examples of the invention will now be described in detail with reference to the accompanying drawings, in which:

Fig. 1 shows a system in accordance with an example of the invention.
Fig. 2 shows a method in accordance with an example of the invention; and
Fig. 3 shows a general computer architecture suitable for implementing the processor used in the system.

DETAILED DESCRIPTION OF THE EMBODIMENTS

The invention provides a system for determining a risk level of a pollen-induced allergy of a user. The system comprises an input for receiving information relating to a pollen level in a location, an input for receiving information relating to a particulate matter level in the location and an input for receiving information relating to a sensitivity of a user to pollen allergen. The system further comprises a processor which is adapted to determine the risk level by taking account of the information relating to a pollen level, the information relating to a particulate matter level and the information relating to a sensitivity of the user to pollen allergen. A user interface communicates the risk level in the location to the user.

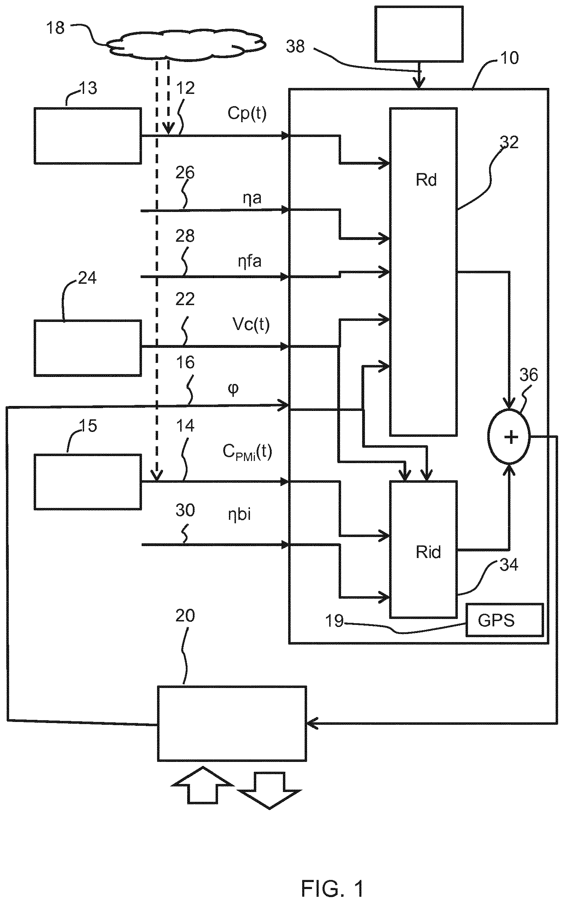

Fig. 1 shows a system in accordance with an example of the invention. The system comprises a processor 10 which receives various inputs in order to determine a risk level of a pollen-induced allergy of a user.

The inputs comprise an input 12 for receiving information relating to a pollen level in a location, in particular a pollen concentration $C_p(t)$, an input 14 for receiving information relating to a particulate matter level in the location, in particular a particulate matter concentration $C_{PMi}(t)$ and an input 16 for receiving information $\phi$ relating to a sensitivity of a user to pollen allergen.

The input 12 for receiving information relating to a pollen level in a location and the input 14 for receiving information relating to a particulate matter level in the location may receive the information wirelessly from remote data sources such as pollen and particulate concentration mapping and forecasts. In an alternative embodiment, the information may be inputted manually by the user. The pollen and particulate level information may be obtained or supplemented by on-board sensors, such as a pollen sensor 13 and a particle sensor 15.

Various pollen sensors are known and are capable of providing real-time information on the pollen level in a location. A pollen sensor may be implemented as an optical particle sensor for detecting a particular particle size range. In order to distinguish between different types of pollen particles, particle size and/or particle size distribution may be obtained. Accurate pollen sensing systems for consumer use are not highly reliable, and the system may rely additionally (or even principally or solely) on externally obtained information, for example obtained over the internet 18 based on location information. This information may derive from a geographical pollen map covering the location of the system. The information for receiving information relating to a pollen level may be obtained from multiple sources. Location may be obtained automatically by GPS (shown as unit 19) or other location determining systems, or else location may be inputted manually.

The information relating to a pollen level may relate to a general pollen concentration or it may provide information relating to individual pollen types or groups of pollen type. The risk level may thus be a general risk level or it may relate to specific pollen types.

Various particle sensors are known and are capable of providing real-time information on the particulate matter level in a location. The particle sensor may also be implemented as an optical sensor for detecting a particular particle size range.

As for the pollen level information, the system may rely principally on externally obtained information, for example obtained over the internet 18 based on location information. This information may derive from a geographical particle pollutant map covering the location of the system. The information for receiving information relating to a particle level may also be obtained from multiple sources.

The information relating to a particulate matter level may relate to all particles below a threshold size, or information may be provided in respect of different particle size ranges. To measure concentrations in different particle size ranges, multiple particle sensors may be used, for example with filters to provide size selectivity, or else an optical particle sensor may provide a set of outputs, for example by ramping a threshold level used during the detection process. The risk level may thus be a general risk level taking into account the general level of particular pollution, or it may take into account different particle sizes. The particle sensor may be adapted to sense diesel exhaust particles (DEP) and/or dust particles. The particle sensor or externally received particle pollutant information for example may indicate the concentration of one or more of PM0.1, PM2.5, PM4 or PM10, where PM2.5 means particles having a diameter of 2.5 $\mu$m or less, and so on.

The information relating to a sensitivity of a user to pollen allergen, received at the input 16 may be inputted by the user, using a user interface 20. The user can consult known scoring systems such as the "Total Nasal Symptom Score" and rate themselves as, for example, "very sensitive", "sensitive", "mild" or "insensitive".

In a further embodiment, the processor may be adapted to carry out a learning method to determine the sensitivity of the user. For example, the processor can record the various parameters (such as pollen level, particulate matter level etc.) and the user can input to the user interface 20 at periodic times whether they are experiencing symptoms or not. By recognition of patterns of both the parameters and symptom over time, the processor 10 is therefore able to create a model for estimating the sensitivity of the user.

Multiple sensitivity measures may be used, for different pollen types, in particular if the pollen sensing information takes into account different pollen types. The risk level is communicated to the user by the user interface 20. The user interface 20 may comprise a mobile device (telephone or tablet) with which the processor communicates wirelessly. The risk level may be communicated via sound or vibration alert, or by SMS or other means of visual display. Alternatively, the system may include a physical output device such as a display or speaker.

The minimum information processed by the system comprises information relating to a particulate matter level, to a pollen level and to the pollen sensitivity of the user. These three information sources enable a direct risk level $R_d$ to be determined which relates to direct passage of pollen into the user's airway, as well as an indirect risk level $R_{id}$ which relates to the transport of pollen allergen to the user on a particulate matter carrier. The direct risk depends primarily on the pollen level and the user sensitivity, whereas the indirect risk depends primarily on the particulate matter level as well as the user sensitivity. Using these sources of information, an overall risk level may be determined, by making assumptions about the relative risk associated with particulate matter compared to the risk associated with direct pollen inhalation. Thus, no other variables are needed for a most basic implementation.

However, the system of Fig. 1 includes various further inputs to enable a more accurate assessment of the overall risk level, tailored to the particular individual. The processor 10 has a further input 22 relating to the breathing rate $V_c(t)$ of the user. This is measured by a breathing sensor 24. The processor 10 is further adapted to take account of the breathing rate in determining the risk level. The breathing sensor 24 may be incorporated into a chest strap or smart watch worn by the user which is preferably capable of communicating wirelessly with the processor 10.

The breathing rate correlates to the amount of air inhaled, and is thus relevant to the amount of pollen inhaled by the user. The breathing rate $V_c(t)$ is a breathing flow rate with respect to time, so its integral over time provides a flow volume which is representative of the air inhalation.

The breathing rate $V_c(t)$ is relevant to the assessment of both the direct risk and the indirect risk.

A further input 26 is for receiving information relating to the probability $\eta_a$ of allergen release from the pollen, and the processor 10 is further adapted to take account of the probability of allergen release in determining the risk level. This information can be stored in a memory of the system or received wirelessly from remote data sources. In an alternative embodiment, the information may be inputted manually by the user.

The probability of allergen release from the pollen is influenced by various factors such as the species of pollen, weather conditions and pollution level. This probability can be pre-determined by experiment.

The system comprises a further input 28 for receiving information $\eta_{fa}$ relating to the probability of free allergen release - in other words allergen that is released from the pollen and does not bind to particulate matter. The processor 10 is further adapted to take account of the probability of pollen allergen binding in determining the risk level. These two probability values are relevant to the direct risk factor.

The probability of pollen allergen binding to the particulate matter or being free is influenced by various factors such as the type and size of the allergen and particulate matter. This probability can be pre-determined by experiment.

The direct allergy risk comprises a first component which simply follows from the pollen concentration, the amount of air breathed in and the user sensitivity. A second component relates to the pollen allergen which is released from the pollen and then breathed in (not on a particulate matter carrier). This component is related to the product $\eta_a$ x $\eta_{fa}$, since this provides the probability of pollen allergen being released and also remaining free.

A further input 30 is for receiving information $\eta_{bi}$ relating to the probability that pollen allergens bind to particles. The information can be stored in a memory of the system or received wirelessly from remote data sources. In an alternative embodiment, the information may be inputted manually by the user. This probability can be pre-determined by experiment.

The processor determines the risk level as the sum of the two components $R_d$ and $R_{id}$ mentioned above. Fig. 1 shows a first processing unit 32 for deriving the direct risk $R_d$ and a second processing unit 34 for deriving the indirect risk $R_{id}$.

$R_d$ is calculated according to the following formula:

$$R_d = \int_0^t (1 + \eta_a \cdot \eta_{fa}) \cdot C_p(t) \cdot V_c(t) \cdot \varphi \cdot dt$$

The first component is the integral over time of $C_P(t)V_c(t)\phi$ and it relates to the direct passage of allergen to the user carried by the pollen. The second component is the product of this with the combined probability $\eta_{fa}\eta_a$. This relates to the free released allergen (which is additional to the pollen concentration measured as Cp(t)).

$R_{id}$ is the indirect risk calculated according to the following formula.

$$R_{id} = \int_0^t \sum [C_{PMi}(t) \cdot \eta_{bi}] \cdot V_c(t) \cdot \varphi \cdot dt$$

.

This relates to the released allergen which has become bound to particulate pollutants.

As explained above:

$C_p(t)$ is the pollen concentration over time.
$V_c(t)$ is the breath volume of the user over time.
$\eta_a$ is the probability of allergen release from the pollen.
$\eta_{fa}$ is the probability of free allergen release.
$\phi$ is the sensitivity of the user to pollen allergen.
t is the exposure time.
$C_{PMi}(t)$ is the concentration of particles with diameter below i (unit: $\mu$m) over time.
$\eta_{bi}$ is the probability that pollen allergens bind to these particles.

The processor 10 comprises an adder 36 for adding the two components and provides the result to the user interface 20 so that it can be provided to the user. The measure may be a dimensionless value, for example normalized to a range of 0 to 1.

As mentioned above, the risk level is also dependent on the weather and seasonal conditions. The system thus preferably has an input 38 for receiving climate information, which may, for example, be used to modify the probability of allergen release from the pollen $\eta_a$.

There may be local sensors, for example a timer and location source (e.g. GPS) enables season information to be obtained, and temperature and moisture sensors may enable weather conditions to be obtained. Of course external weather sources may be accessed to obtain the climate information.

In the example above, the pollen level and particulate matter level are provided as concentration levels, namely as a measure of particle numbers per unit volume. However, the risk level may only need to be presented with a few possible levels (e.g. no risk, low risk, medium risk, high risk). Accordingly, the calculations to reach these levels do not need to process highly accurate information. Thus, the analog equations above may be replaced with more simple equivalents. For example the pollen concentration may simply be one of a small set of pollen level indicators, such as a scale of 1 to 5. Similarly, the particulate matter concentration may also be one of a small set of levels.

These inputs may be processed by more simple combinatory equations or by accessing look up tables which map combinations of inputs to a determined risk level. Not all inputs need to be taken into account. For example, a default breathing pattern may be assumed, or this may be modified based on motion sensing (to detect a level of physical exertion) rather than by measuring actual breathing rate. The probability values may be absolute constants or they may themselves vary in dependence on other parameters. For example the probability values may themselves depend on the concentration levels obtained or even concentration distribution information.

The example above shows one value for each of the probability measures. However, there may be different probability levels for different pollen types, and for different particle size distributions.

The invention may be implemented as a computer program for processing inputs from remote sources, or the system may combine some or all of the sensing functions using hardware components with the required signal processing.

In use of the device, the pollen and particle level information is measured and/or received from external sources. Optionally taking into account of the breathing rate of the user, the risk is determined as explained above and presented to the user. The user can then decide if it is safe to go to certain places, or wear a mask or take medicine in advance. It can help a user to build up greater knowledge of their own pollen sensitivity.

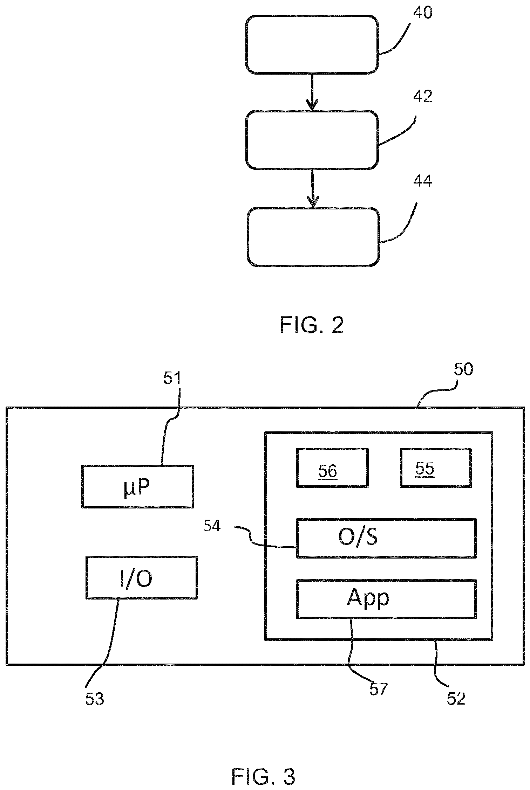

Fig. 2 shows a method of the invention.

In step 40, information relating to a pollen level in a location, a particulate matter level in the location and a sensitivity of a user to pollen allergen is received. Further information may also be received, such as a breathing rate of the user, information relating to the probability of allergen release from the pollen and information relating to the probability of pollen allergen binding to the particulate matter.

In step 42, the risk level of a pollen-induced allergy of a user is determined by taking account of the information relating to a pollen level, the information relating to a particulate matter level and the information relating to a sensitivity of the user to pollen allergen.

Optionally, further information such as a breathing rate of the user, information relating to the probability of allergen release from the pollen and information relating to the probability of pollen allergen binding to the particulate matter may be taken into account in determining the risk level.

In step 44, the risk level is communicated to the user.

The system described above makes use of a processor for processing data.

Fig. 3 illustrates an example of a computer 50 for implementing the processor described above.

The computer 50 includes, but is not limited to, PCs, workstations, laptops, PDAs, palm devices, servers, storages, and the like. Generally, in terms of hardware architecture, the computer 50 may include one or more processors 51, memory 52, and one or more I/O devices 53 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 51 is a hardware device for executing software that can be stored in the memory 52. The processor 51 can be virtually any custom made or commercially available processor, a central processing unit (CPU), a digital signal processor (DSP), or an auxiliary processor among several processors associated with the computer 50, and the processor 51 may be a semiconductor based microprocessor (in the form of a microchip) or a microprocessor.

The memory 52 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 52 may incorporate electronic, magnetic, optical, and/or other types of storage media. Note that the memory 52 can have a distributed architecture, where various components are situated remote from one another, but can be accessed by the processor 51.

The software in the memory 52 may include one or more separate programs, each of which comprises an ordered listing of executable instructions for implementing logical functions. The software in the memory 52 includes a suitable operating system (O/S) 54, compiler 55, source code 56, and one or more applications 57 in accordance with exemplary embodiments.

The application 57 comprises numerous functional components such as computational units, logic, functional units, processes, operations, virtual entities, and/or modules.

The operating system 54 controls the execution of computer programs, and provides scheduling, input-output control, file and data management, memory management, and communication control and related services.

Application 57 may be a source program, executable program (object code), script, or any other entity comprising a set of instructions to be performed. When a source program, then the program is usually translated via a compiler (such as the compiler 55), assembler, interpreter, or the like, which may or may not be included within the memory 52, so as to operate properly in connection with the operating system 54. Furthermore, the application 57 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 53 may include input devices such as, for example but not limited to, a mouse, keyboard, scanner, microphone, camera, etc. Furthermore, the I/O devices 53 may also include output devices, for example but not limited to a printer, display, etc. Finally, the I/O devices 53 may further include devices that communicate both inputs and outputs, for instance but not limited to, a network interface controller (NIC) or modulator/demodulator (for accessing remote devices, other files, devices, systems, or a network), a radio frequency (RF) or other transceiver, a telephonic interface, a bridge, a router, etc. The I/O devices 53 also include components for communicating over various networks, such as the Internet or intranet.

When the computer 50 is in operation, the processor 51 is configured to execute software stored within the memory 52, to communicate data to and from the memory 52, and to generally control operations of the computer 50 pursuant to the software. The application 57 and the operating system 54 are read, in whole or in part, by the processor 51, perhaps buffered within the processor 51, and then executed.

When the application 57 is implemented in software it should be noted that the application 57 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A system for determining a risk level of a pollen-induced allergy of a user, comprising:

    an input (12) for receiving information relating to a pollen level in a location;
    an input (14) for receiving information relating to a particulate matter level in the location;
    an input (16) for receiving information relating to a sensitivity of a user to pollen allergen;
    a further input (30) for receiving information relating to the probability of pollen allergen binding to the particulate matter;
    a processor (10) which is adapted to determine the risk level by taking account of the information relating to a pollen level, the information relating to a particulate matter level, the information relating to a sensitivity of the user to pollen allergen, and the probability of pollen allergen binding to the particulate matter; and
    a user interface (20) for communicating the risk level in the location to the user.

2. The system of claim 1, comprising a pollen sensor (13) for generating the information relating to the pollen level in the location.

3. The system of claim 1 or 2, comprising a particle sensor (15) for generating the information relating to a particulate matter level in the location.

4. The system of any of claims 1 to 3, comprising a breathing sensor (24) for determining a breathing rate of the user, and wherein the processor (10) is further adapted to take account of the breathing rate in determining the risk level.

5. The system of any of claims 1-4, comprising a further input (26) for receiving information relating to the probability of allergen release from the pollen, and wherein the processor (10) is further adapted to take account of the probability of allergen release in determining the risk level.

6. The system of any of claims 1-5, wherein the processor (10) is adapted to determine the risk level based on a pollen concentration, breathing characteristics of the user, a probability of allergen release from the pollen, a probability of free allergen release, a sensitivity of the user to pollen allergen, a particulate matter concentration, and said probability that pollen allergens bind to the particulate matter.

7. The system of claim 6, wherein the processor (10) is adapted to determine the risk level as the sum of two components $R_d$ and $R_{id}$;

   wherein $R_d$ is the direct risk calculated according to the following formula:

$$R_d = \int_0^t (1 + \eta_a \cdot \eta_{fa}) \cdot C_p(t) \cdot V_c(t) \cdot \varphi \cdot dt$$

   wherein $R_{id}$ is the indirect risk calculated according to the following formula:

$$R_{id} = \int_0^t \sum [C_{PMi}(t) \cdot \eta_{bi}] \cdot V_c(t) \cdot \varphi \cdot dt$$

   wherein

   $C_p(t)$ is the pollen concentration over time,
   $V_c(t)$ is the breath volume of the user over time,
   $\eta_a$ is the probability of allergen release from the pollen,
   $\eta_{fa}$ is the probability of free allergen release,
   $\phi$ is the sensitivity of the user to pollen allergen,
   t is the exposure time,
   $C_{PMi}(t)$ is the concentration of particles with diameter below i over time, and
   $\eta_{bi}$ is the probability that pollen allergens bind to these particles.

8. A computer implemented method for determining a risk level of a pollen-induced allergy of a user, comprising:

   receiving information relating to a pollen level in a location;
   receiving information relating to a particulate matter level in the location;
   receiving information relating to a sensitivity of a user to pollen allergen;
   receiving information relating to the probability of pollen allergen binding to the particulate matter;
   determining the risk level by taking account of the information relating to a pollen level, the information relating to a particulate matter level, the information relating to a sensitivity of the user to pollen allergen, and the probability of pollen allergen binding to the particulate matter; and
   communicating the risk level in the location to the user.

9. The method of claim 8, comprising determining a breathing rate of the user, and taking account of the breathing rate in determining the risk level.

10. The method of claim 8 or 9, comprising receiving information relating to the probability of allergen release from the pollen, and taking account of the probability in determining the risk level.

11. The method of any of claims 8-10, comprising determining the risk level based on a pollen concentration, breathing characteristics of the user, a probability of allergen release from the pollen, a probability of free allergen release, a sensitivity of the user to pollen allergen, a particulate matter concentration, and the probability that pollen allergens bind to the particulate matter.

**12.** The method of any of claims 8-11, comprising determining the risk level as sum of two components $R_d$ and $R_{id}$;

wherein $R_d$ is the direct risk calculated according to the following formula:

$$R_d = \int_0^t (1 + \eta_a \cdot \eta_{fa}) \cdot C_p(t) \cdot V_c(t) \cdot \varphi \cdot dt \quad ;$$

wherein $R_{id}$ is the indirect risk calculated according to the following formula:

$$R_{id} = \int_0^t \sum [C_{PMi}(t) \cdot \eta_{bi}] \cdot V_c(t) \cdot \varphi \cdot dt \quad ;$$

wherein

$C_p(t)$ is the pollen concentration over time,
$V_c(t)$ is the breath volume of the user over time,
$\eta_a$ is the probability of allergen release from the pollen,
$\eta_{fa}$ is the probability of free allergen release,
$\phi$ is the sensitivity of the user to pollen allergen,
t is the exposure time,
$C_{PMi}(t)$ is the concentration of particles with diameter below i over time, and
$\eta_{bi}$ is the probability that pollen allergens bind to these particles.

**13.** A computer program which is adapted, when said program is run on a computer, to implement the method of any of claims 8 to 12.

**Patentansprüche**

**1.** System zur Bestimmung des Risikoniveaus einer polleninduzierten Allergie eines Benutzers, Folgendes umfassend:

einen Eingang (12) zum Empfangen von Informationen in Bezug auf einen Pollenflug an einem Ort; einen Eingang (14) zum Empfangen von Informationen in Bezug auf einen Feinstaubgehalt an dem Ort;
einen Eingang (16) zum Empfangen von Informationen bezüglich der Empfindlichkeit eines Benutzers gegenüber Pollenallergene;
einen weiteren Eingang (30) zum Empfangen von Informationen, die sich auf die Wahrscheinlichkeit der Bindung von Pollenallergenen an die Partikel beziehen; einen Prozessor (10), der so beschaffen ist, dass er den Risikograd unter Berücksichtigung der Informationen, die sich auf einen Pollenflug beziehen, der Informationen, die sich auf einen Partikelgehalt beziehen, der Informationen, die sich auf eine Empfindlichkeit des Benutzers gegenüber Pollenallergenen beziehen, und der Wahrscheinlichkeit der Bindung von Pollenallergenen an die Partikel bestimmt; und eine Benutzerschnittstelle (20), um dem Benutzer den Risikograd am Ort mitzuteilen.

**2.** Das System nach Anspruch 1 umfasst einen Pollensensor (13) zur Erzeugung der Informationen über den Pollenflug an dem Ort.

**3.** Das System nach Anspruch 1 oder 2 umfasst einen Partikelsensor (15) zur Erzeugung von Informationen über den Feinstaubgehalt an dem Ort.

**4.** System nach einem der Ansprüche 1 bis 3, das einen Atmungssensor (24) zur Bestimmung der Atemfrequenz des Benutzers umfasst, und wobei der Prozessor (10) ferner so ausgelegt ist, dass er die Atemfrequenz bei der Bestimmung des Risikoniveaus berücksichtigt.

**5.** System nach einem der Ansprüche 1-4, umfassend einen weiteren Eingang (26) zum Empfangen von Informationen bezüglich der Wahrscheinlichkeit der Allergenfreisetzung aus den Pollen, und wobei der Prozessor (10) ferner dazu ausgelegt ist, die Wahrscheinlichkeit der Allergenfreisetzung bei der Bestimmung des Risikoniveaus zu berücksichtigen.

**6.** System nach einem der Ansprüche 1 bis 5, wobei der Prozessor (10) so ausgelegt ist, dass er das Risikoniveau auf der Grundlage einer Pollenkonzentration, der Atemcharakteristik des Benutzers, einer Wahrscheinlichkeit der Allergenfreisetzung aus den Pollen, einer Wahrscheinlichkeit der Freisetzung freier Allergene, einer Empfindlichkeit des Benutzers gegenüber Pollenallergenen, einer Partikelkonzentration und der Wahrscheinlichkeit, dass Pollenallergene an die Partikel binden, bestimmt.

**7.** System nach Anspruch 6, wobei der Prozessor (10) so ausgelegt ist, dass er das Risikoniveau als die Summe von zwei Komponenten $R_d$ und $R_{id}$; bestimmt; wobei $R_d$ das direkte Risiko ist, das nach der folgenden Formel berechnet wird:

$$R_d = \int_0^t (1 + \eta_a \cdot \eta_{fa}) \cdot C_p(t) \cdot V_c(t) \cdot \varphi \cdot dt \quad ;$$

wobei $R_{id}$ das indirekte Risiko ist, das nach der folgenden Formel berechnet wird:

$$R_{id} = \int_0^t \sum [C_{PMi}(t) \cdot \eta_{bi}] \cdot V_c(t) \cdot \varphi \cdot dt \quad ;$$

wobei

$C_p(t)$ entspricht der Pollenkonzentration über einen bestimmten Zeitraum,
$V_c(t)$ ist das Atemvolumen des Benutzers über einen bestimmten Zeitraum,
$\eta_a$ ist die Wahrscheinlichkeit der Allergenfreisetzung aus den Pollen,
$\eta_{fa}$ ist die Wahrscheinlichkeit der Freisetzung des Allergens,
$\phi$ ist die Empfindlichkeit des Benutzers gegenüber Pollenallergenen,
$t$ ist die Belichtungszeit,
$C_{PMi}(t)$ ist die Konzentration von Partikeln mit einem Durchmesser unter i über einen bestimmten Zeitraum, und
$\eta_{bi}$ ist die Wahrscheinlichkeit, dass sich Pollenallergene an diese Partikel binden.

**8.** Ein computerimplementiertes Verfahren zur Bestimmung des Risikoniveaus einer polleninduzierten Allergie eines Benutzers,

Folgendes umfassend: Empfangen von Informationen bezüglich eines Pollenflugs an einem Ort; Empfangen von Informationen bezüglich eines Feinstaubgehalts an dem Ort; Empfangen von Informationen bezüglich einer Empfindlichkeit eines Benutzers gegenüber Pollenallergenen; Empfangen von Informationen bezüglich der Wahrscheinlichkeit der Bindung von Pollenallergenen an die Feinstaubpartikel; Bestimmen des Risikoniveaus unter Berücksichtigung der Informationen bezüglich des Pollenflugs, der Informationen bezüglich des Feinstaubflugs, der Informationen bezüglich der Empfindlichkeit des Benutzers gegenüber dem Pollenallergen und der Wahrscheinlichkeit der Bindung des Pollenallergens an die Feinstaubpartikel; und Übermittlung des Risikoniveaus an den Nutzer vor Ort.

**9.** Verfahren nach Anspruch 8, umfassend die Bestimmung der Atemfrequenz des Benutzers und die Berücksichtigung der Atemfrequenz bei der Bestimmung des Risikoniveaus.

**10.** Verfahren nach Anspruch 8 oder 9, bei dem Informationen über die Wahrscheinlichkeit der Allergenfreisetzung aus den Pollen empfangen werden und die Wahrscheinlichkeit bei der Bestimmung des Risikoniveaus berücksichtigt wird.

**11.** Verfahren nach einem der Ansprüche 8-10, umfassend die Bestimmung des Risikoniveaus auf der Grundlage einer Pollenkonzentration, der Atemcharakteristika des Benutzers, einer Wahrscheinlichkeit der Allergenfreisetzung aus den Pollen, einer Wahrscheinlichkeit der Freisetzung freier Allergene, einer Empfindlichkeit des Benutzers gegenüber Pollenallergenen, einer Partikelkonzentration und der Wahrscheinlichkeit, dass Pollenallergene an die Partikel binden.

**12.** Verfahren nach einem der Ansprüche 8 bis 11, bei dem das Risikoniveau als Summe von zwei Komponenten $R_d$ und $R_{id}$ bestimmt wird;

wobei $R_d$ das direkte Risiko ist, das nach der folgenden Formel berechnet wird:

$$R_d = \int_0^t (1 + \eta_a \cdot \eta_{fa}) \cdot C_p(t) \cdot V_c(t) \cdot \varphi \cdot dt \quad ;$$

wobei $R_{id}$ das indirekte Risiko ist, das nach der folgenden Formel berechnet wird:

$$R_{id} = \int_0^t \sum [C_{PMi}(t) \cdot \eta_{bi}] \cdot V_c(t) \cdot \varphi \cdot dt \quad ;$$

wobei

$C_p(t)$ entspricht der Pollenkonzentration über einen bestimmten Zeitraum,
$V_c(t)$ ist das Atemvolumen des Benutzers über einen bestimmten Zeitraum,
$\eta_a$ ist die Wahrscheinlichkeit der Allergenfreisetzung aus den Pollen,
$\eta_{fa}$ ist die Wahrscheinlichkeit der Freisetzung des Allergens,
$\phi$ ist die Empfindlichkeit des Benutzers gegenüber Pollenallergenen,
$t$ ist die Belichtungszeit,
$C_{pMi}(t)$ ist die Konzentration von Partikeln mit einem Durchmesser unter i über einen bestimmten Zeitraum, und
$\eta_{bi}$ ist die Wahrscheinlichkeit, dass sich Pollenallergene an diese Partikel binden.

**13.** Ein Computerprogramm, das auf einem geeignetem Computer das Verfahren nach einem der Ansprüche 8 bis 12 durchführt.

## Revendications

**1.** Un système pour déterminer un niveau de risque d'une allergie au pollen d'un utilisateur comprend:

une entrée (12) pour recevoir des informations concernant le niveau de pollen dans un lieu;
une entrée (14) pour recevoir des informations concernant le niveau de matière particulaire dans un lieu;
une entrée (16) pour recevoir des informations concernant la sensibilité d'un utilisateur à une allergie au pollen;
une autre entrée (30) pour recevoir des informations concernant la probabilité d'allergène de pollen se liant à la matière particulaire;
un processeur (10) qui est adapté pour déterminer le niveau de risques en prenant en compte les informations concernant le niveau de pollen, les informations concernant le niveau de matière particulaire, les informations concernant la sensibilité de l'utilisateur à l'allergène de pollen, et la probabilité d'allergène de pollen se liant à la matière particulaire; et
une interface utilisateur (20) pour communiquer sur les niveaux de risques du lieu où se trouve l'utilisateur.

**2.** Le système de la revendication 1 comprend un capteur de pollen (13) pour générer des informations concernant le niveau de pollen du lieu.

**3.** Le système de la revendication 1 ou 2 comprend un capteur de particules (15) pour générer des informations concernant le niveau de matière particulaire du lieu.

**4.** Le système selon l'une quelconque des revendications 1 à 3, comprend un détecteur de respiration (24) pour déterminer la fréquence respiratoire de l'utilisateur, où le processeur (10) est également adapté pour prendre en compte la fréquence respiratoire dans la détermination du niveau de risque.

5. Le système selon l'une quelconque des revendications 1 à 4, comprend une autre entrée (26) pour recevoir des informations concernant la probabilité de libération d'allergène à partir du pollen, et où le processeur (10) est également adapté pour prendre en compte la probabilité de libération des allergènes dans la détermination du niveau de risque.

6. Le système selon l'une quelconque des revendications 1 à 5, où le processeur (10) est adapté pour déterminer le niveau de risque à partir de la concentration de pollen, des caractéristiques respiratoires de l'utilisateur, la probabilité de libération d'allergène à partir du pollen, une probabilité de libération exemptée d'allergènes, la sensibilité de l'utilisateur à l'allergène du pollen, une concentration de matière particulaire, et ladite probabilité que les allergènes de pollen soient liés à la matière particulaire.

7. Le système de la revendication 6, où le processeur (10) est adapté pour déterminer le niveau de risque comme la somme de deux composantes $R_d$ et $R_{id}$; où $R_d$ représente le risque direct calculé selon la formule suivante:

$$R_d = \int_0^t (1 + \eta_a \cdot \eta_{fa}) \cdot C_p(t) \cdot V_c(t) \cdot \varphi \cdot dt \quad ;$$

où $R_{id}$ représente le risque indirect calculé selon la formule suivante:

$$R_{id} = \int_0^t \sum [C_{PMi}(t) \cdot \eta_{bi}] \cdot V_c(t) \cdot \varphi \cdot dt \quad ;$$

où

$C_p(t)$ représente la concentration de pollen au fil du temps,
$V_c(t)$ représente le volume respiratoire de l'utilisateur au fil du temps,
$\eta_a$ représente la probabilité de libération d'allergène à partir du pollen,
$\eta_{fa}$ représente la probabilité de libération exemptée d'allergène,
$\phi$ représente la sensibilité de l'utilisateur à l'allergène de pollen,
t représente le temps d'exposition,
$C_{PMi}(t)$ représente la concentration de particules avec un diamètre en dessous de i au fil du temps, et
$\eta_{bi}$ représente la probabilité que les allergènes de pollen soient liés à ces particules.

8. Une méthode exécutée par un ordinateur pour déterminer un niveau de risque d'une allergie au pollen d'un utilisateur comprend:

la réception d'informations concernant le niveau de pollen dans un lieu;
la réception d'informations concernant le niveau de matière particulaire dans un lieu;
la réception d'informations concernant la sensibilité d'un utilisateur à un allergène de pollen;
la réception d'information concernant la probabilité que l'allergène au pollen soit lié à la matière particulaire;
la détermination du niveau de risques en prenant en compte les informations concernant le niveau de pollen, les informations concernant le niveau de matière particulaire, les informations concernant la sensibilité de l'utilisateur à l'allergène de pollen, et la probabilité d'allergène de pollen se liant à la matière particulaire; et
communiquer sur les niveaux de risques du lieu où se trouve l'utilisateur.

9. La méthode de la revendication 8 comprend la détermination de la fréquence respiratoire de l'utilisateur, et en prenant en compte la fréquence respiratoire dans la détermination du niveau de risque.

10. La méthode selon la revendication 8 ou 9 comprend la réception d'informations concernant la probabilité de libération d'allergène à partir du pollen, et prendre en compte la probabilité de libération des allergènes dans la détermination du niveau de risque.

11. La méthode selon l'une quelconque des revendications 8-10, comprend la détermination du niveau de risque à partir de la concentration de pollen, des caractéristiques respiratoires de l'utilisateur, la probabilité de libération d'allergène à partir du pollen, une probabilité de libération exemptée d'allergènes, la sensibilité de l'utilisateur à l'allergène du pollen, une concentration de matière particulaire, et ladite probabilité que les allergènes de pollen soient liés à la matière particulaire.

**12.** La méthode selon l'une quelconque des revendications 8-11, comprend la détermination du niveau de risque comme la somme de deux composantes $R_d$ et $R_{id}$; où $R_d$ représente le risque direct calculé selon

$$R_d = \int_0^t (1 + \eta_a \cdot \eta_{fa}) \cdot C_p(t) \cdot V_c(t) \cdot \varphi \cdot dt$$

la formule suivante:

; où $R_{id}$ représente le risque indirect calculé selon la formule suivante :

$$R_{id} = \int_0^t \sum [C_{PMi}(t) \cdot \eta_{bi}] \cdot V_c(t) \cdot \varphi \cdot dt \quad ;$$

où

$C_p(t)$ représente la concentration de pollen au fil du temps,
$V_c(t)$ représente le volume respiratoire de l'utilisateur au fil du temps
$\eta_a$ représente la probabilité de libération d'allergène à partir du pollen
$\eta_{fa}$ représente la probabilité de libération exemptée d'allergène
$\phi$ représente la sensibilité de l'utilisateur à l'allergène de pollen,
$t$ représente le temps d'exposition,
$C_{PMi}(t)$ représente la concentration de particules avec un diamètre en dessous de i au fil du temps, et
$\eta_{bi}$ représente la probabilité que les allergènes de pollen soient liés à ces particules.

**13.** Un programme informatique qui est adapté, lorsque ledit programme est exécuté sur un ordinateur, pour appliquer la méthode des revendications 8 à 12.

FIG. 1

FIG. 2

FIG. 3

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2001029535 A
- US 2015242586 A